# EUROPÄISCHE PATENTSCHRIFT

(11) **EP 2 445 552 B1**
(45) Veröffentlichungstag und Bekanntmachung des Hinweises auf die Patenterteilung: **14.10.2015**
(21) Anmeldenummer: 09779934.0
(22) Anmeldetag: 24.06.2009
(51) Int. Cl.: A61M 5/315, A61M 5/31, A61M 5/24, A61M 5/50

(54) **VERABREICHUNGSGERÄT MIT PRIMING FUNKTION**
ADMINISTERING DEVICE HAVING A PRIMING FUNCTION
APPAREIL D ADMINISTRATION À FONCTION D AMORÇAGE

(43) Veröffentlichungstag der Anmeldung: 02.05.2012
(73) Patentinhaber: Tecpharma Licensing AG, 3401 Burgdorf (CH)
(72) Erfinder: SELZ, Anjan, CH-3014 Bern (CH); RETTENBACHER, Manfred, CH-5616 Meisterschwanden (CH); KIRCHHOFER, Fritz, CH-3454 Sumiswald (CH)
(74) Vertreter: Schwabe - Sandmair - Marx
(86) Internationale Anmeldenummer: PCT/EP2009/057934
(87) Internationale Veröffentlichungsnummer: WO 2010/149214

(56) Entgegenhaltungen:
- EP-A1- 0 373 321
- EP-A1- 1 101 505
- WO-A1-2005/039676
- WO-A2-00/62839
- WO-A2-97/01362
- US-A1- 2003 158 523

## Beschreibung

Die Erfindung betrifft ein Verabreichungsgerät für ein flüssiges Medikament, das insbesondere eine flüssige Formulierung sein kann, die FSH oder eine FSH Variante enthält. Als flüssige Medikamente werden im Sinne der Erfindung nicht nur Flüssigkeiten im engeren Sinn angesehen, sondern auch pasten- und gelartige Medikamente, solange sich derartige Medikamente nur vergleichbar einer Flüssigkeit fördern lassen. Das Verabreichungsgerät ist vorzugsweise ein Injektionsgerät für die Verabreichung mittels einer infundierenden Injektionsnadel, bevorzugt für eine subkutane Injektion, kann grundsätzlich aber auch ein Injektionsgerät für eine nadellose Verabreichung oder beispielsweise auch ein Inhalationsgerät sein. Injektionsgeräte in Form sogenannter Injektionspens sind besonders bevorzugte Ausführungsbeispiele.

Moderne Injektionspens ermöglichen die genaue Dosierung des Medikaments, individuell auch durch den jeweiligen Patienten selbst. Die durch die freie Auswahl der Dosis erhöhte Flexibilität ermöglicht den Einsatz in Therapien, in denen die Patienten sich das jeweilige Medikament selbst verabreichen, der sogenannten Selbstverabreichung. Gerade in der Selbstverabreichung sind eine hohe Bedienungssicherheit und ein hoher Bedienungskomfort gefordert. Die Geräte sollen Fehlbedienungen von Hause aus verhindern. Eine Fehlerquelle ist der Einschluss von Luft im Medikamentenreservoir. Wird diese Luft nicht vor der Verabreichung entfernt, besteht die Gefahr, dass nicht das Medikament in der eingestellten Dosis, sondern das Medikament gemeinsam mit der eingeschlossenen Luft entsprechend der eingestellten Dosis verabreicht wird. Das Medikamentenreservoir wird daher vor der Verabreichung entlüftet, ein Vorgang der allgemein unter dem Begriff des "Primen" bekannt ist. Bei einem Großteil der Geräte bleibt das Primen dem Gefühl des Patienten überlassen, der hierfür an einem Dosierglied eine kleine Dosis einstellt, das Gerät mit der Nadel nach oben hält und das eingestellte Primingvolumen durch Betätigung des Geräts ins Freie ausschüttet. Andere Geräte, beispielsweise ein aus der EP 0 927 058 A1 bekanntes Gerät, sind eigens mit Priming-Mechanismen ausgestattet, die allerdings einen erheblichen konstruktiven Aufwand und daher nennenswert erhöhte Kosten mit sich bringen.

Die US 2003/0158523 A1 offenbart ein Injektionsgerät mit einem Gehäuse, in dem eine Mehrkammerampulle aufgenommen ist, und einem Dosier- und Förderglied, mittels dem eine pulverförmige und eine flüssige Komponente des Medikaments abgemischt und das abgemischte Medikament verabreicht werden kann. Das Dosier- und Förderglied ist zur Einstellung einer zu verabreichenden Dosis um eine Längsachse drehbeweglich und zum Abmischen des Medikaments und Ausschütten der eingestellten Dosis in die axiale Vortriebsrichtung beweglich. Das Dosier- und Förderglied wirkt mit dem Gehäuse derart zusammen, dass es nach dem Abmischen und noch vor dem Einstellen der Dosis einen Priminghub ausführen muss. Zur Realisierung dieser Ablaufsteuerung ragen vom Innenumfang des Gehäuses nach radial innen Vorsprünge ab, und das Dosier- und Förderglied weist axial nebeneinander einen Gewindeabschnitt, einen Primingabschnitt und einen Deblockierabschnitt auf. Beim Abmischen greifen die Vorsprünge in das Gewinde des Gewindeabschnitts ein, so dass das Dosier- und Förderglied in dem so gebildeten Gewindeeingriff in das Gehäuse geschraubt wird. Am Ende der Schraubbewegung gelangt das Dosier- und Förderglied im Bereich seines Primingabschnitts in Drehrichtung auf Anschlag gegen einen der Vorsprünge des Gehäuses, so dass eine weitere Drehbewegung des Dosier- und Förderglieds in Einschraubrichtung verhindert wird. Dieser in die eine Drehrichtung wirksame Verdrehblockiereingriff wird durch einen axialen Priminghub des Dosier- und Förderglieds gelöst. Nach Ausführung des Priminghubs kann das Dosier- und Förderglied relativ zum Gehäuse wieder verdreht und dadurch die zu verabreichende Dosis eingestellt werden.

Die WO 00/62839 A2 beschreibt ein Injektionsgerät mit einem Dosier- und Förderglied, das nach Ausführung eines kurzen Priminghubs manuell gegen die Vortriebsrichtung zurückgezogen wird. Durch das Zurückziehen wird ein Verdrehsicherungseingriff des Dosier- und Förderglieds gelöst, so dass dieses in der zurückgezogenen Position verdrehbar und dadurch die zu verabreichende Dosis einstellbar ist.

Wegen der Notwendigkeit der Kostensenkung werden der Selbstverabreichung immer mehr Therapien erschlossen. Ein Beispiel ist die Stimulierung der Eierstöcke und daraus folgend durch Befruchtung der stimulierten Eizellen der Schwangerschaft durch Hormonbehandlung. So beschreibt beispielsweise die EP 1 188 444 B1 flüssige Formulierungen auf der Basis von FSH (follicle stimulating hormone) und FSH Varianten. Um FSH und FSH Varianten auch in der Selbstverabreichung verabreichen zu können, wird die flüssige Formulierung, das Medikament, konserviert. Dem Wirkstoff, FSH oder einer FSH Variante, wird in flüssiger Lösung ein Konservierungsmittel beigemischt. Unkonserviert bestünde die Gefahr der Kontamination des FSH oder eine FSH Variante enthaltenden Medikaments, so dass es nach Kontakt mit Luft innerhalb weniger Stunden, am besten sofort, verabreicht werden müsste. Das Medikament wird daher meist in konservierter Form in größeren Behältnissen abgegeben, aus denen die Patienten ihr Verabreichungsgerät aufziehen, d.h. dessen Reservoir mit dem konservierten Medikament befüllen können. Alternativ erhält der Patient zwei Behälter, einen mit einer nicht konservierten Formulierung und den anderen mit dem Konservierungsmittel, so dass der Patient sich das konservierte Medikament selbst mischen und im danach konservierten Zustand über einen längeren Zeitraum lagern kann. Das Konservierungsmittel übt über die konservierende Wirkung hinaus auf den Wirkstoff, FSH oder FSH Varianten, allenfalls negativen Einfluss auf die Stabilität des Wirkstoffs und erhöht auch den Preis des fertigen, konservierten Medikaments. Das Aufziehen oder Mischen des Verabreichungsgeräts aus einem steril geschlossenen größeren Behältnis, das eine konservierte oder nicht konservierte Formulierung enthält, ist umständlich. Zudem erfordert es ein Aufziehen exakt der zu verabreichenden Dosis.

Es ist eine Aufgabe der Erfindung, ein Verabreichungsgerät zu schaffen, das insbesondere in Therapien mit Selbstverabreichung eingesetzt werden kann, im Aufbau einfach und preiswert ist, aber dennoch eine sichere Gewähr für die Verabreichung genau der erforderlichen Dosis verbunden mit einem der Selbstverabreichung adäquaten Bedienungskomfort bietet. Das Gerät soll insbesondere für die Verabreichung einer flüssigen, nicht konservierten Formulierung, die FSH oder eine FSH Variante enthält, geeignet sein.

Die Erfindung hat ein Verabreichungsgerät zum Gegenstand, das ein Gehäuse, ein Reservoir für ein flüssiges Medikament, ein Dosierglied zum Einstellen einer zu verabreichenden Dosis des Medikaments und eine Fördereinrichtung mit einem Förderglied zum Ausschütten der eingestellten Dosis umfasst. Das Gehäuse kann unmittelbar das Reservoir bilden, falls beispielsweise eine Spritze das Gehäuse oder einen Teil des Gehäuses bildet. Das Reservoir kann insbesondere aber auch ein in dem Gehäuse aufgenommenes Behältnis, vorzugsweise in Form einer Karpule, sein. Das Dosierglied ist zum Einstellen der Dosis relativ zum Gehäuse beweglich. Die Bewegung, die das Dosierglied bei der Auswahl der Dosis ausführt, und die Beweglichkeit, die es für die Auswahl der Dosis relativ zum Gehäuse haben muss, werden im Folgenden als Dosierbewegung und Dosierbeweglichkeit bezeichnet. Die Fördereinrichtung kann insbesondere als Linearhub-Fördereinrichtung gebildet sein und dementsprechend einen axial beweglichen Kolben aufweisen, der das Reservoir an einem Ende steril abdichtet und im Falle einer Vortriebsbewegung Medikament durch einen Auslass des Reservoirs verdrängt und dadurch ausschüttet. Das genannte Förderglied kann den Kolben bilden. Bevorzugter bildet das Förderglied eine auf den Kolben wirkende Kolbenstange oder ist ein im Förderstrang noch weiter aufwärts angeordnetes Förderglied, das aber letztlich auf den Kolben oder ein anderes unmittelbar auf das im Reservoir befindliche Medikament wirkendes Förderglied abtreibt. Bei dem Förderglied handelt es sich in bevorzugten Ausführungen jedenfalls um eines, das eine Vortriebsbewegung, im folgenden auch als Ausschüttbewegung bezeichnet, ausführt.

In bevorzugten Ausführungen besteht das Verabreichungsgerät nur aus den genannten Komponenten, nämlich dem Gehäuse, dem Reservoir, dem Förderglied und dem Dosierglied, wobei das Förderglied und das Dosierglied vorzugsweise gemeinsam in einem Stück geformt oder alternativ durch zwei separat geformte und bevorzugterweise unbeweglich fest miteinander verbundene Teile gebildet werden. Bei Formung in einem einzigen Stück oder dem Fügen zu einer nur im Ganzen beweglichen Struktur werden die funktionalen Komponenten "Dosierglied" und "Förderglied" nachfolgend auch als "Dosier- und Förderglied" bezeichnet. Das Reservoir kann wie gesagt vom Gehäuse selbst gebildet werden, das in derartigen Ausführungen insbesondere als Spritze gebildet sein oder ein spritzenartiges Gehäuseteil aufweisen kann. Umfasst die Fördereinrichtung wie bevorzugt einen Kolben, so wird es bevorzugt, wenn der Kolben bereits von Hause aus im Reservoir angeordnet ist, also vom Hersteller im Reservoir angeordnet wird und nicht vom Patienten. Bei dem Kolben handelt es sich vorzugsweise um einen einfachen Stopfen ohne Kolbenstange. Die Kolbenstange oder ein vergleichbares anderes Förderglied wirkt nur in einem axialen Druckkontakt während der Förderung gegen die Rückseite des Kolbens, um diesen im Reservoir in die Vortriebsrichtung zu bewegen. Alternativ kann eine Kolbenstange aber auch beim Zusammenbau des Geräts formschlüssig mit einem einfachen Stopfen, der von Hause aus keine Kolbenstange aufweist, verbunden werden. Der Zusammenbau des Geräts wird vorzugsweise vom Gerätehersteller oder dem Medikamentenhersteller vorgenommen, so dass der Patient oder auch ein die Therapie gegebenenfalls begleitender Arzt das fertige Gerät mit dem bereits mit dem Medikament gefüllten Reservoir erhält und lediglich noch die Verabreichung als solche vornehmen muss.

Das Dosierglied ist in einem Ausgangszustand des Verabreichungsgeräts, in dem der Patient das Gerät vorzugsweise erhält, in einem lösbaren Dosierblockiereingriff in Richtung der Dosierbewegung blockiert und kommt erst am Ende einer der Entlüftung des Reservoirs dienenden Primingbewegung des Förderglieds von der Blockierung frei, automatisch ohne weiteres Zutun des Patienten, der nur die Primingbewegung bewirken muss. Das Förderglied führt die Primingbewegung in eine Richtung quer zur Richtung der Dosierbewegung aus. Die Richtung der Primingbewegung des Förderglieds ist auch die Vortriebsrichtung bei der Ausschüttbewegung. Die Vortriebsrichtung wird im Folgenden auch als axiale Richtung bezeichnet, sie erfolgt vorzugsweise längs einer zentralen Längsachse des Geräts. Nach der Erfindung ist das Dosierglied durch eine in die Vortriebsrichtung wirkende Kraft gegen eine elastische Rückstellkraft in die Vortriebsrichtung aus dem Dosierblockiereingriff bewegbar.

Das Verabreichungsgerät verfügt somit über eine Ablaufsteuerung, die einen bestimmten Ablauf bei der Verabreichung erzwingt und in diesem Sinne steuert. Aufgrund des Dosierblockiereingriffs muss das Reservoir vor dem Einstellen der Dosis entlüftet werden. Die Ablaufsteuerung ist in bevorzugter Ausführung ferner so verwirklicht, dass vor dem Ausschütten des Medikaments die Dosis eingestellt werden muss, so dass die Verabreichung nur in der Sequenz "Primen - Dosieren - Ausschütten" ablaufen kann.

Das Dosierglied ist für die Einstellung der Dosis quer zu der Vortriebsrichtung beweglich. Eine Dosierdrehbeweglichkeit um eine Rotationsachse, die insbesondere mit einer zentralen Längsachse des Geräts zusammenfallen kann, wird besonders bevorzugt. In derartigen Ausführungen ist das Dosierglied im Dosierblockiereingriff in Umfangsrichtung um die Rotationsachse gegen einen Verdrehanschlag auf Anschlag, drückt also bei dem Versuch der Einstellung einer Dosis in Bezug auf die Rotationsachse in tangentialer Richtung gegen den Verdrehanschlag. Vorzugsweise ist das Dosierglied und die Rotationsachse hin und her drehbeweglich und in Bezug auf beide Drehrichtungen in einem Dosierblockiereingriff, drückt also beim Versuch der Verdrehung in beide Drehrichtungen gegen jeweils einen Verdrehanschlag.

Das Dosierglied ist in Vortriebsrichtung des Förderglieds längs des Verdrehanschlags aus dem Dosierblockiereingriff beweglich. Bevorzugterweise wird es bei der Primingbewegung des Förderglieds und noch bevorzugter durch die Primingbewegung automatisch längs des Verdrehanschlags oder des bevorzugt linken und rechten Verdrehanschlags aus dem Dosierblockiereingriff bewegt. Bevorzugten Ausführungen entspricht es, wenn das Dosierglied mit dem Förderglied so gekoppelt ist, dass es die Primingbewegung des Förderglieds mitmacht. Das Dosierglied und das Förderglied können insbesondere in einem Stück gemeinsam geformt oder als separate Teile geformt und fest miteinander gefügt sein.

Die Erfindung eröffnet auch Wege für eine sichere Verabreichung von flüssigen Medikamenten, deren Wirkstoff(e) an Luft der Gefahr der Kontamination unterliegt, wie insbesondere Formulierungen, die als wesentlichen Wirkstoff FSH oder eine FSH Variante enthalten. Dabei ist es ein besonderes Anliegen, den Einsatz von Konservierungsmitteln zu vermeiden, nicht zuletzt im Hinblick auf negative Auswirkungen auf den oder die jeweiligen Wirkstoff(e), aber auch auf die zusätzlichen Kosten, die durch die Beimischung von Konservierungsmitteln entstehen. Nach der Erfindung wird das Reservoir mit dem nicht konservierten flüssigen Medikament an den Patienten abgegeben, vorzugsweise in Form eines kompletten Verabreichungsgeräts oder eines mit einfachen Handgriffen montierbaren Geräteteils. Das nicht konservierte, bei Anwesenheit von Luft kontaminationsgefährdete Medikament ist in dem bis unmittelbar vor Verabreichung steril geschlossenen Reservoir sicher aufbewahrt, so dass es in diesem Zustand über einen Zeitraum von mehreren Tagen oder durchaus auch Monaten oder Jahren aufbewahrt werden kann. Die Sterilität wird erst unmittelbar vor Gebrauch vom Patienten oder einem verabreichenden Arzt aufgehoben.

Die Erfindung betrifft insbesondere ein erfindungsgemäßes Verabreichungsgerät mit Ablaufsteuerung, dessen Reservoir steril geschlossen ist und ein flüssiges Medikament in Form beispielsweise einer flüssigen Formulierung von FSH oder einer FSH Variante enthält. Die Verwendung von Karpulen oder Spritzen, die ein nicht konserviertes Medikament, beispielsweise FSH oder eine nicht konservierte FSH Variante, in sterilem und daher auch ohne Konservierungsmittel haltbaren Zustand enthalten, ist von Vorteil Weitere Medikamente, die nach der Erfindung in nicht konserviertem Zustand in einem steril verschlossenen Reservoir des Verabreichungsgeräts und somit praktisch zeitlich unbegrenzt im Handel, beim Arzt oder auch bereits beim jeweiligen Patienten aufbewahrt werden können, sind beispielsweise Neuroleptica (Fluphenazini decanoas), gefäßerweiternde Mittel (Adrenalinum), Blutprodukte (Etamsylat, Epoetin alfa, Filgrastim (G-CSF), Nadroparinum calcium, Desmopressini acetas), Medikamente gegen rheumatische Erkrankungen (Methotrexat, Etanerceptum), Onkologica (Cladribinum, Interferonum humanum gamma-1b ADN) und Medikamente gegen Infektionskrankheiten (Herpes simplex Typ1/Typ2, humanes Immunglobulin). In Klammern sind übliche oder bevorzugte Wirkstoffe für die jeweilige Medikamentengruppe angegeben.

Ein FSH basiertes Medikament enthält vorzugsweise eine alpha- und eine beta-Untereinheit. Die Proteine für die verwendbaren Formulierungen können durch verschiedene Verfahren gewonnen werden. Vorzugsweise ist das einzusetzende FSH ein Heterodimer, das eine α-Untereinheit und eine β-Untereinheit umfasst, wie sie in der EP 1 188 444 A1 näher beschrieben werden. Vorzugsweise wird mit der erfindungsgemäßen Vorrichtung eine Formulierung von FSH und/oder einer FSH-Variante in einem wässrigen Lösungsmittel verabreicht. Der Ausdruck "wässriges Lösungsmittel" bezieht sich auf ein flüssiges Lösungsmittel, das Wasser enthält. Wässrige Lösungsmittelsysteme können nur aus Wasser bestehen oder können aus Wasser und einem oder mehreren mischbaren Lösungsmitteln bestehen und können weitere gelöste Bestandteile (Solute) enthalten, wie beispielsweise Zucker oder andere Hilfsstoffe. Die herkömmlich verwendeten mischbaren Lösungsmittel sind die kurzkettigen organischen Alkohole, wie Methanol, Ethanol, Propanol, kurzkettige Ketone, wie Aceton, und Polyalkohole, wie Glycerin. Bevorzugt besteht das Lösungsmittelsystem nur aus Wasser für Injektionszwecke. Hilfsstoffe können ausgewählt sein aus Isotonizitätsmitteln, Konservierungsstoffen, Puffersystemen, insbesondere Phosphatpuffern, Thioetherverbindungen wie Methionin als Antioxidationsmittel, Dispergiermitteln bzw. Emulgatoren wie Poloxameren und deren Gemischen. Die Formulierung enthält keine Konservierungsstoffe, insbesondere keinen Konservierungsstoff der Gruppe, bestehend aus Phenol, m-Cresol, Chlorcresol, einem Paraben, das ausgewählt ist aus Methyl-, Ethyl-, Propyl- oder Butylparaben, Benzalkoniumchlorid, Benzethoniumchlorid, Natriumdehydroacetat, Benzylakohol und Thiomerosal. Bevorzugt ist weiterhin, wenn die Formulierung frei ist von Poly-, Oligo- und Dicarbonsäuren sowie Glycin und/oder Glycerin.

Insbesondere für die Verabreichung von nicht konservierten flüssigen Medikamenten, wie die Verabreichung flüssiger Formulierungen von FSH oder einer FSH Variante, ist es von Vorteil, wenn im steril geschlossenen Reservoir nur eine Medikamentenmenge aufbewahrt wird, die höchstens so groß wie eine Tagesdosis, noch bevorzugter wie eine Maximaldosis (Monodosis) ist, in der das betreffende Medikament in einer einzigen Verabreichung von höchstens einigen Minuten Dauer, vorzugsweise einer einzigen Injektion, verabreicht wird. Das Reservoir und somit auch das Verabreichungsgerät sind daher vorzugsweise ein Reservoir und ein Verabreichungsgerät für eine Monodosis des Medikaments. Die Maximaldosis für Medikamente auf der Basis von beispielsweise FSH oder einer FSH Variante ist typischerweise 300 IU, in Ausnahmefällen 500 IU, gemessen über eine Vielzahl von Patienten. Die im Reservoir befindliche Medikamentenmenge entspricht im Falle solch eines Medikaments daher in bevorzugten Ausführungen höchstens einer Dosis von 500 IU, bevorzugter höchstens 300 IU. Da eine Mehrzahl der Patienten mit deutlich geringeren Dosen pro Einzelverabreichung auskommt, beispielsweise mit höchstens 200 IU oder auch nur höchstens 100 IU, kann die Füllmenge in solch einer Therapie vorteilhafterweise auch höchstens dieser Dosis entsprechen.

In einer Weiterentwicklung werden die Verabreichungsgeräte jeweils mit einem Reservoir angeboten, das eine dem tatsächlichen Bedürfnis des jeweiligen Patienten genauer angepasste Füllmenge aufweist. So können baugleiche Verabreichungsgeräte beispielsweise in zwei, drei oder mehr unterschiedlichen Füllmengenvarianten zum Einsatz gelangen. Im Falle beispielsweise einer FSH-basierten Therapie können Verabreichungsgeräte der gleichen Bauart angeboten werden, die sich im Wesentlichen nur hinsichtlich der Füllmenge ihres Reservoirs voneinander unterscheiden, beispielsweise ein Gerät mit einem Reservoir, das 300 IU des Medikaments, ein weiteres Gerät, das 200 IU des gleichen Medikaments und noch ein Gerät, das 100 IU des Medikaments enthält, jeweils im sterilen Zustand. Es kann auch eine nochmalige Unterteilung in Zwischenstufen vorgenommen werden, beispielsweise in Form einer vierten Füllmengenvariante mit einem Reservoir, dass 150 IU des Medikaments im sterilen Zustand enthält. Das Gerät ist vorzugsweise jeweils für den einmaligen Gebrauch konzipiert, als Einwegartikel, und kann vorteilhafterweise über den Hausmüll entsorgt werden. Erst unmittelbar vor der Verabreichung wird das Medikament im bis zu diesem Zeitpunkt steril geschlossenen Reservoir mit der äußeren Umgebung in Kontakt gebraucht.

Für den sterilen Verschluss des Reservoirs kommen insbesondere zwei Varianten in Betracht. In der ersten Variante bildet eine Karpule das Reservoir, die für die Verabreichung einen Auslass aufweist, der von einem durchstechbaren Septum steril verschlossen wird. Das Medikament ist in derartigen Ausführungen zwischen dem Septum und einem axial beweglichen Kolben steril eingeschlossen. Mit einem derartigen Reservoir kommt eine Nadeleinheit zum Einsatz mit einem Nadelhalter und einer den Nadelhalter durchragenden Injektionsnadel. Der Nadelhalter dient der Halterung der Injektionsnadel und der Herstellung des Fluidanschlusses der Nadel an das Reservoir. Für den Anschluss kann unmittelbar das Reservoir oder vorzugsweise das Gehäuse einen Verbindungsabschnitt im Bereich des Auslasses des Reservoirs aufweisen. Der Verbindungsabschnitt und ein Verbindungsabschnitt des Nadelhalters sind kooperierend ausgebildet, so dass bei Herstellung der mechanischen Verbindung zwischen dem Nadelhalter und dem Verbindungsabschnitt des Reservoirs oder des Gehäuses die Injektionsnadel das Septum durchsticht. Nach dem Durchstechen ist der Innenraum des Reservoirs über die Nadel mit der Umgebung verbunden. Im Anschluss an die Herstellung dieser Fluidverbindung wird möglichst ohne Verzug das Medikament verabreicht. Anschließend wird das Verabreichungsgerät oder zumindest ein das vollständig oder teilweise entleerte Reservoir enthaltender Teil des Geräts, gegebenenfalls auch nur das Reservoir, entsorgt. In der zweiten Variante bildet eine Spritze oder spritzenähnliches Gebilde das Reservoir. Im Unterschied zur ersten Variante weist das Reservoir der zweiten Variante an seinem distalen Ende bereits eine Injektionsnadel auf, so dass von Hause aus bereits die Fluidverbindung zwischen dem Reservoir und einem stromabwärtigen Auslass der Injektionsnadel besteht. Für den sterilen Verschluss sorgt ein externes Dichtelement, das den Nadelauslass steril verschließt. Das Dichtelement wird unmittelbar vor Gebrauch abgezogen.

Auch für Therapien mit nicht konserviertem Medikament oder einem für nur eine einzige Verabreichung befüllten Reservoir, einer Monodosis, ist es von Vorteil, wenn das Verabreichungsgerät über die Fähigkeit der Dosierbarkeit verfügt, also das zuvor genannte Dosierglied aufweist. Auf diese Weise kann trotz einer optionalen Vielfalt von Varianten hinsichtlich der Füllmenge für jeweils nur eine einzige Verabreichung in jeder Variante nochmals die Dosis genauer eingestellt werden. Es kann mit anderen Worten durch die Bereitstellung mehrerer Füllmengenvarianten die Medikamentenmenge über viele Patienten gesehen insgesamt verringert werden, nämlich im Vergleich zu Ausführungen, in denen das Reservoir jeweils die Maximaldosis enthält, die innerhalb der Therapie überhaupt in einer einzigen Verabreichung ausgeschüttet wird. Andererseits kann der einzelne Patient innerhalb der passend gewählten Füllmengenvariante nochmals individuell die auf ihn zugeschnittene Dosis einstellen und auch nur diese sich verabreichen.

Es wird bevorzugt, wenn das Primen und anschließend das Ausschütten der eingestellten Dosis richtungsgleiche Bewegungen sind, vorzugsweise axiale Bewegungen in die Vortriebsrichtung eines das Medikament verdrängenden Kolbens. So verhindert der Dosierblockiereingriff im Ausgangszustand Dosierbewegungen des Dosierglieds, ermöglicht jedoch den Priminghub, durch den das Dosierglied am Ende aus dem Dosierblockiereingriff und gegen einen Priminganschlag in Druckkontakt, vorzugsweise in axialen Druckkontakt gelangt. Nach Ausführung des Priminghubs, insbesondere im Kontakt mit dem Priminganschlag, ist das Dosierglied quer zur Richtung der Primingbewegung beweglich, um die gewünschte Dosis einstellen zu können. In bevorzugten Ausführungen sind das Dosierglied und das Förderglied in Bezug auf die Richtung des Priminghubs fest miteinander verbunden, beispielsweise in einem Stück geformt. Sollten die beiden Glieder diesbezüglich relativ zueinander in alternativen Ausführungen beweglich sein, kann entweder das Dosierglied in den Anschlagkontakt mit dem Priminganschlag gelangen oder, bevorzugter, das Förderglied.

In bevorzugten Ausführungen ist ein Rückhalteelement vorgesehen, mit dem das Förderglied im Verlaufe seiner Ausschüttbewegung in einen Rückhalteeingriff gelangt, der eine Bewegung des Förderglieds gegen die Vortriebsrichtung blockiert oder zumindest erschwert. Einer Blockierung wird zwar der Vorzug gegeben, ein lösbarer Rückhalteeingriff ist jedoch ebenfalls von Vorteil. Um in den Rückhalteeingriff zu gelangen, bewegt sich das Förderglied bei der Ausschüttbewegung vorteilhafterweise über das Rückhalteelement hinweg, so dass ein hörbares oder fühlbares Klick-Geräusch entsteht. Das Rückhalteelement und ein entsprechendes Eingriffsgegenelement des Förderglieds sind relativ zueinander vorteilhafterweise so angeordnet, dass sich der Rückhalteeingriff automatisch erst am Ende der Ausschüttbewegung einstellt, so dass dem Patienten hörbar oder fühlbar signalisiert wird, dass die Ausschüttung vollständig vorgenommen wurde.

Um die Herstellkosten auch von Verabreichungsgeräten so niedrig wie möglich zu halten, die die Einstellung der Dosis erlauben, schlägt die Erfindung vor, das Gehäuse, und die Funktionen des Primens und Förderns und, soweit verwirklicht, auch des Dosierens durch insgesamt nur zwei oder drei getrennt geformte Teile zu verwirklichen. Falls das Reservoir ein im Gehäuse aufgenommenes Behältnis, vorzugsweise eine Karpule ist, kommt das Behältnis noch dazu, gemeinsam mit einem das Behältnis verschließenden Kolben. Diese Teile können insbesondere im Spritzguss aus Kunststoff geformt sein, wobei ein aufgenommenes Behältnis auch aus Glas gefertigt sein kann. So kann das Gehäuse mit dem Dosierglied bereits alleine sämtliche Dosierfunktionen des Verabreichungsgeräts erfüllen, das Dosierglied und das Gehäuse somit bereits allein eine vollständige Dosiereinrichtung bilden. Das Gehäuse kann auch mit dem Dosierglied im Ausgangszustand in dem Dosierblockiereingriff sein, insbesondere den in bevorzugten Ausführungen vorhandenen Verdrehanschlag unmittelbar bilden. Ein Betätigungselement durch dessen Betätigung die Ausschüttung ausgelöst wird, kann vorteilhafterweise in einem Stück mit dem Förderglied geformt sein. Das gleiche Betätigungselement kann auch in einem Stück mit dem Dosierglied geformt sein und dessen Bedienbereich für die Dosiseinstellung bilden. Falls das Förderglied separat von dem Dosierglied gefertigt ist, sind die beiden Teile im zusammengebauten Zustand des Geräts vorzugsweise axial oder rotatorisch fest miteinander gefügt. Das Wort "oder" wird hier wie auch sonst stets von der Erfindung im üblichen logischen Sinne von "inklusiv oder" verwendet, umfasst also sowohl die Bedeutung von "entweder .... oder" als auch die Bedeutung von "und", soweit sich aus dem jeweils konkreten Zusammenhang nicht ausschließlich nur eine dieser Bedeutungen ergeben kann. Bezogen auf die Fertigung des Dosierglieds und Förderglieds als getrennte Teile bedeutet dies für den gefügten Zustand, dass das Förderglied und das Dosierglied entweder nur axial relativ zueinander unbeweglich oder nur rotatorisch relativ zueinander unbeweglich oder aber, wie bevorzugt, sowohl axial als auch rotatorisch relativ zueinander unbeweglich miteinander gefügt sind. In einer bevorzugten Modifikation bildet ein in einem einzigen Stück geformtes Dosier- und Förderglied eine Kolbenstange, wenigstens ein für die Auswahl der Dosis erforderliches Dosierelement, einen den Ausschütthub begrenzenden Ausschüttanschlag und ein Bedienelement für die Auswahl der Dosis oder die Betätigung zum Ausschütten. Bevorzugt bildet solch ein Dosier- und Förderglied auch noch einen Priminganschlag für die Begrenzung des Priminghubs. Von den drei genannten Formelementen, dem Dosierelement, dem Ausschüttanschlag und dem Priminganschlag, können zwei oder bevorzugt alle drei vom gleichen Formelement gebildet werden. Das Dosierblockierelement kann ebenfalls in einem Stück am Dosier- und Förderglied geformt sein, ebenso ein optionales Dosierrastelement oder eine Dosierraststruktur zur Einstellung der Dosis in diskreten Schritten. Das Dosierblockierelement oder Dosierrastelement oder die Dosierraststruktur kann oder können in bevorzugten Abwandlungen auch separat von dem die genannten verbleibenden Funktionen aufweisenden Dosier- und Förderglied geformt und mit diesem vorzugsweise fest, d. h. translatorisch und rotatorisch unbeweglich gefügt sein.

Ein Verabreichungsgerät, das die Einstellung der Dosis erlaubt, verfügt vorzugsweise über eine Dosisanzeige mit einer Dosisskala und einem Marker, dessen Position relativ zur Dosisskala die eingestellte Dosis anzeigt. Der Marker kann insbesondere von einem Fenster in einem Schalenabschnitt des Gehäuses gebildet werden, durch das die Dosisskala ablesbar ist. Die Dosisskala kann insbesondere auf einer äußeren Umfangsfläche des Dosierglieds gebildet sein, die bei der Einstellung der Dosis unter dem Fenster durchläuft. Anstatt im Gehäuse ein Fenster zu formen, kann das Gehäuse auch bereichsweise oder im Ganzen aus einem transparenten oder transluzenten Material gefertigt sein und einen Marker in Form eines Aufdrucks oder eines abragenden Formelements aufweisen.

In einer Weiterentwicklung kann solch eine Dosisanzeige den Anschlag, vorzugsweise Verdrehanschlag, für die Blockierung des Dosierglieds im Dosierblockiereingriff bilden. Der Dosierblockieranschlag kann insbesondere von einer Seitenwand des Fensters oder von einem als Abragung geformten Marker gebildet werden. Das gleiche Fenster kann noch einen weiteren Anschlag bilden, nämlich einen Anschlag, den das Dosierglied in Richtung der Primingbewegung überwinden muss, um aus dem Dosierblockiereingriff zu gelangen. Dieser optionale weitere Anschlag muss vorteilhafterweise gegen die elastische Rückstellkraft überwunden werden, wobei die Rückstellkraft quer zur Richtung der Primingbewegung weist. Die Rückstellkraft kann insbesondere von einem elastischen Schnapper, einer Biegezunge, bereitgestellt werden, die am Dosierglied geformt ist. In Umkehrung der Verhältnisse kann solch ein Schnapper auch am Gehäuse geformt sein. Gegebenenfalls können auch ein elastischer Schnapper des Dosierglieds und ein elastischer Gegenschnapper des Gehäuses in der geschilderten Weise zusammenwirken, um das Dosierglied lösbar im Dosierblockiereingriff zu halten.

Vorteilhafte Merkmale werden auch in den Unteransprüchen und deren Kombinationen beschrieben.

Nachfolgend werden Ausführungsbeispiele der Erfindung anhand von Figuren erläutert. An den Ausführungsbeispielen offenbar werdende Merkmale bilden jeweils einzeln und in jeder Merkmalskombination die Gegenstände der Ansprüche und auch die vorstehend beschriebenen Ausgestaltungen vorteilhaft weiter. Es zeigen:
- Figur 1: ein Verabreichungsgerät eines ersten Ausführungsbeispiels in einem Ausgangszustand vor einem Primen,
- Figur 2: das Verabreichungsgerät nach dem Primen und Einstellen einer Dosis,
- Figur 3: das Verabreichungsgerät nach dem Ausschütten der eingestellten Dosis,
- Figur 4: einen Längsschnitt des Verabreichungsgeräts im Ausgangszustand,
- Figur 5: einen Längsschnitt des Verabreichungsgeräts nach dem Primen,
- Figur 6: einen Längsschnitt des Verabreichungsgeräts nach dem Ausschütten,
- Figur 7: ein Detail der Figur 4, nämlich einen Dosierblockiereingriff eines Dosierglieds des Verabreichungsgeräts,
- Figur 8: ein Detail der Figur 5, nämlich das aus dem Dosierblockiereingriff bewegte Dosierglied,
- Figur 9: einen Dosierabschnitt des Verabreichungsgeräts in einem Querschnitt und
- Figur 10: ein Detail der Figur 6, nämlich das in einem Rückhalteeingriff befindliche Dosierglied.

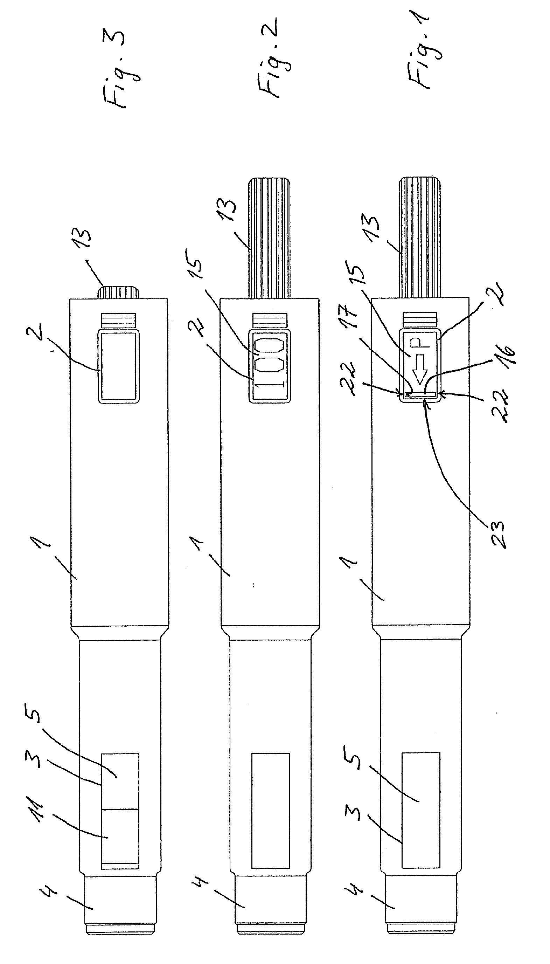

Die Figuren 1 bis 3 zeigen jeweils in der gleichen Seitenansicht ein Verabreichungsgerät eines ersten Ausführungsbeispiels. Bei dem Verabreichungsgerät handelt es sich um einen langgestreckten Injektionspen. Das Verabreichungsgerät ist für den einmaligen Gebrauch bestimmt, für eine einzige Injektion eines flüssigen Medikaments, das insbesondere eine flüssige Formulierung auf der Basis von FSH oder einer FSH Variante für eine Hormonbehandlung zur Stimulierung der Eierstöcke und daraus folgend durch Befruchtung der stimulierten Eizellen der Schwangerschaft sein kann. Das flüssige Medikament, d.h. die flüssige Formulierung, ist nicht konserviert, enthält also keine Konservierungsmittel. Handelt es sich wie bevorzugt um eine Formulierung, die FSH oder eine FSH Variante enthält, muss das Medikament nach Kontakt mit der Umgebungsluft daher innerhalb weniger Minuten, allenfalls innerhalb weniger Stunden, verabreicht werden.

Figur 1 zeigt das Verabreichungsgerät in einem Ausgangszustand vor der Benutzung. In diesem Zustand erhält der Patient das Gerät. Das Gerät umfasst ein hülsenförmiges Gehäuse 1 mit einem vorderen, distalen Gehäuseabschnitt, in dem ein das Medikament in sterilem Zustand enthaltendes Reservoir 5 aufgenommen ist, und einen hinteren, proximalen Gehäuseabschnitt, in dem Strukturen geformt sind, die mit einer Dosier- und Fördereinrichtung des Geräts zusammenwirken, um dem Patienten eine individuelle Auswahl einer zu verabreichenden Dosis des Medikaments zu ermöglichen. Der proximale Gehäuseabschnitt kann bei der Verabreichung auch als Griffteil dienen. Von der Dosier- und Fördereinrichtung ist ein Bedienelement 13 erkennbar, das in proximaler Richtung am proximalen Ende aus dem Gehäuse 1 ragt.

Im Gehäuse 1 sind ein Dosisanzeigefenster 2 und ein Reservoirfenster 3 geformt. Durch das Fenster 2 ist die eingestellte Dosis ablesbar, und durch das Fenster 3 ist das Reservoir 5 erkennbar. Das Gehäuse 1 weist am distalen Ende einen Verbindungsabschnitt 4 auf, an dem eine Nadeleinheit befestigt werden kann. Die Nadeleinheit wird erst unmittelbar vor der Verabreichung befestigt, um bis zu diesem Zeitpunkt die Sterilität des Medikaments zu gewährleisten.

Figur 1 zeigt das Verabreichungsgerät wie gesagt im Ausgangszustand mit steril verschlossenem Reservoir 5, genauer gesagt im Lagerungszustand, in dem das Gerät über einen längeren Zeitraum von wenigstens mehreren Monaten gelagert werden kann, da der luftdichte, sterile Verschluss des Reservoirs 5 die Haltbarkeit auch von nicht konservierten Medikamenten sicherstellt. Um sich das Medikament zu verabreichen, schließt der Patient unmittelbar vor der Verabreichung eine Nadeleinheit mit Injektionsnadel an dem Gehäuse 1 an, nämlich in dessen Verbindungsabschnitt 4. Bei dem Anschließen durchsticht die Injektionsnadel mit einem proximalen Nadelabschnitt ein Septum, das einen Auslass des Reservoirs 5 luftdicht und steril verschließt. Das Medikament steht nach dem Durchstechen über die Injektionsnadel mit der Umgebung in Verbindung und muss im Falle eines bevorzugt nicht konservierten Medikaments auf der Basis von FSH oder einer FSH Variante innerhalb kurzer Zeit, am besten unverzüglich verabreicht werden.

Das vom Hersteller gefüllte Reservoir 5 enthält eine Maximaldosis des Medikaments, die so bemessen ist, dass sie über eine größere Gruppe potentieller Patienten gesehen für jeden dieser Patienten ausreicht. Personen, die pro Verabreichung mit einer geringeren Dosis auskommen, können sich diese geringere Dosis selbst einstellen. Das Gerät wird nach Gebrauch mit der im Reservoir 5 entsprechend noch verbliebenen Restmenge des Medikaments entsorgt. Ferner verfügt das Gerät über die Fähigkeit des Primens, also der Entlüftung des Reservoirs 5. Durch das Primen wird im Reservoir 5 möglicherweise enthaltenes freies Gas, insbesondere Stickstoff oder Luft, aus dem Reservoir 5 verdrängt. Luft kann in der Herstellung beim Befüllen des Reservoirs 5 oder bei dem Anschluss der Injektionsnadel 7 in das Reservoir 5 gelangen. Diese Luft oder gegebenenfalls ein anderes Gas soll zum einen nicht verabreicht werden, verfälscht zum anderen aber auch die Dosis, da die verabreichte Dosis um die Menge des ohne das Primen im Reservoir 5 befindlichen Gases von der eingestellten Dosis abweichen würde.

Das Verabreichungsgerät umfasst eine Fördereinrichtung 10 mit einem ersten Förderglied 11 und einem zweiten Förderglied 12. Das erste Förderglied 11 ist ein im Reservoir 5 beweglich aufgenommener Kolben 11, der das Reservoir 5 an einem Ende steril abdichtet und im Reservoir 5 in eine Vortriebsrichtung längs einer zentralen Längsachse A des Geräts in Richtung auf den Auslass beweglich ist. Die Injektionsnadel 7 erstreckt sich ebenfalls axial auf der Achse A. Das zweite Förderglied 12 bildet eine Kolbenstange. Das Förderglied 12 ist mit dem Kolben 11 lediglich in axialem Druckkontakt, drückt also den Kolben 11 bei Betätigung des Geräts durch losen Druckkontakt axial in Richtung auf den Auslass des Reservoirs 5.

Das Förderglied 12 bildet gemeinsam mit dem Gehäuse 1 auch eine Dosiereinrichtung. Das Förderglied 12 wird im folgenden daher als Dosier- und Förderglied 12 bezeichnet. Das Dosier- und Förderglied 12 ist zur Erfüllung der Dosierfunktion relativ zu dem Gehäuse 1 um die Achse A drehbeweglich, führt also bei der Einstellung der Dosis eine Dosierdrehbewegung um die Achse A aus. Zur Erfüllung der Förderfunktion ist es längs der Achse A in die Vortriebsrichtung translatorisch beweglich. Ferner bildet das Dosier- und Förderglied 12 mit einem proximalen Endabschnitt das Betätigungselement 13. Am Betätigungselement 13 nimmt der Patient die Einstellung der Dosis vor und bewirkt durch Ausübung einer Druckkraft in die Vortriebsrichtung auch die Ausschüttbewegung des Dosier- und Förderglieds 12. Zur Erfüllung der unterschiedlichen Funktionen, nämlich des Primens, des Dosierens und des Förderns bzw. Ausschüttens, weist das Dosier- und Förderglied 12 mehrere Strukturelemente auf, insbesondere ein erstes Dosierelement 14 für das Dosieren, das gleichzeitig auch einen Ausschüttanschlag bildet und somit selbst sowohl eine Dosierfunktion als auch eine Ausschüttfunktion erfüllt. Ein Dosierblockierelement 16 ist ein weiteres dieser Strukturelemente.

Das erste Dosierelement 14 wirkt bei dem Einstellen der Dosis mit mehreren zweiten Dosierelementen 24ᵢ zusammen, die um die Längsachse A verteilt angeordnet und in einem Dosierabschnitt 1a des Gehäuses 1 an dessen Mantelinnenfläche geformt sind. Die Dosierelemente 24ᵢ, mit i = 1, 2, 3......n, sind axiale Führungen für das erste Dosierelement 14, das mit diesen Führungen 24ᵢ als Eingriffselement zusammenwirkt. Die Dosierelemente bzw. Führungen 24ᵢ weisen unterschiedliche axiale Längen auf, wobei diese Längen jeweils mit einer einstellbaren Dosis korrespondieren. Die Dosierelemente 24ᵢ sind beispielhaft am Innenumfang des Dosierabschnitts 1a als axiale Sacknuten gebildet. Die Sacknuten sind an ihren proximalen Enden offen, so dass das erste Dosierelement 14 entsprechend der Drehwinkelposition des Dosier- und Förderglieds 12 in eine dieser Nuten 24ᵢ in die Vortriebsrichtung einfahren und im Verlaufe eines Ausschütthubs in der betreffenden Nut 24ᵢ bis zu deren distalen Ende in die Vortriebsrichtung bewegt werden kann. Das Dosierelement 14 bildet wie bereits erwähnt in Doppelfunktion einen Ausschüttanschlag, indem es am Ende des Ausschütthubs des Dosier- und Förderglieds 12 gegen einen Ausschüttanschlag 21 des Gehäuses 1, den im Beispiel das distale Ende der jeweiligen Sacknut 24ᵢ bildet, in axialen Anschlagkontakt gelangt. Die Länge des Ausschütthubs entspricht somit der Länge des in Abhängigkeit von der eingestellten Dosis mit dem Dosierelement 14 zusammenwirkenden Dosierelements 24ᵢ.

Das Dosier- und Förderglied 12 bildet mit dem Gehäuse 1 eine Dosierrasteinrichtung. An einem äußeren Umfang des Dosier- und Förderglieds 12 ist hierfür eine um die Rotationsachse A erstreckte Dosierraststruktur 19 geformt. Bei einer Drehbewegung des Dosier- und Förderglieds 12 gleitet ein an dem Gehäuse 1 geformtes, elastisch nachgiebiges Dosierrastelement 29 in Form eines radial nachgiebigen Schnappers über die Dosierraststruktur 19. Die Dosierraststruktur 19 ist in der Art einer Außenverzahnung gebildet, die beispielhaft unmittelbar an der Mantelaußenfläche des Dosier- und Förderglieds 12 vorzugsweise umlaufend um die Achse A geformt ist und deren Teilung mit der Teilung der zweiten Dosierelemente 24ᵢ korrespondiert, so dass das Dosierrastelement 29 jeweils in eine Vertiefung der Dosierraststruktur 19 eingreift, wenn das Dosierelement 14 gerade in axialer Flucht mit einem der Dosierelemente 24ᵢ ist. In Umkehrung der Verhältnisse könnte auch eine Dosierraststruktur an der Mantelinnenfläche des Gehäuses 1 und ein elastisch nachgiebiges Dosierrastelement am Dosier- und Förderglied 12 geformt sein.

Im Ausführungsbeispiel sind alle funktionalen Strukturelemente entweder am Gehäuse 1 oder am Dosier- und Förderglied 12 geformt. In einer ebenfalls bevorzugten Modifikation können das Dosierblockierelement 16 und die Dosierraststruktur 19 auch getrennt von dem Rest des Dosier- und Förderglieds 12 geformt werden, insbesondere an einem Hülsenteil entsprechend dem Hülsenteil des Ausführungsbeispiels, das den im vorstehenden Absatz genannten äußeren Umfang bildet. Solch ein getrennt geformtes Hülsenteil mit dem Dosierblockierelement 16 und der Dosierraststruktur 19 wäre allerdings bevorzugt unbeweglich mit dem das Gehäuse 1 durchragenden, die Kolbenstange bildenden Teil des Dosier- und Förderglieds 12 fest gefügt. Im Ergebnis würde ein gefügtes Dosier- und Förderglied 12 erhalten werden, das der Form nach dem des Ausführungsbeispiels entspräche.

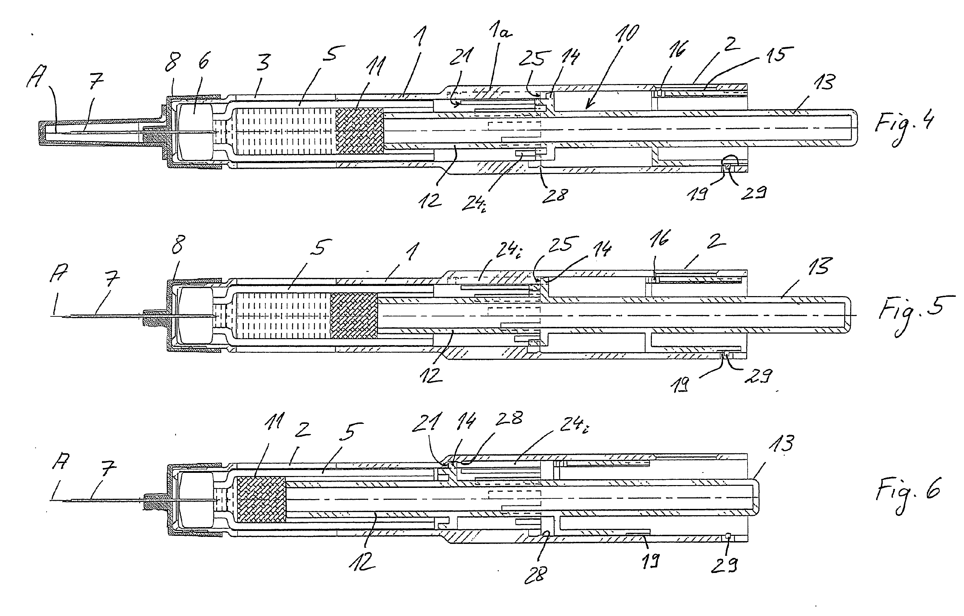

Im Ausgangszustand des Verabreichungsgeräts (Figuren 1 und 4) befindet sich das Dosier- und Förderglied 12 in einem Dosierblockiereingriff, in dem es an einer Drehbewegung um die Achse A gehindert ist. Der Dosierblockiereingriff wird mittels des Dosierblockierelements 16 bewirkt, das ebenfalls an dem Dosier- und Förderglied 12 geformt ist. Das Dosierblockierelement 16 ist mit dem Gehäuse 1 in dem Dosierblockiereingriff. Das Gehäuse 1 bildet hiefür einen Verdrehanschlag 22 als Blockiergegenelement. Der Dosierblockiereingriff wird auf besonders einfache Weise dadurch gebildet, dass das Dosierblockierelement 16 im Ausgangszustand, in dem das Dosier- und Förderglied 12 seine in Bezug auf die Vortriebsrichtung hinterste Position relativ zum Gehäuse 1 und zum Reservoir 5 einnimmt, in das Dosisanzeigefenster 2 ragt, so dass dessen linke und rechte Seitenwand im und gegen den Uhrzeigersinn um die Achse A jeweils als ein Verdrehanschlag 22 wirken.

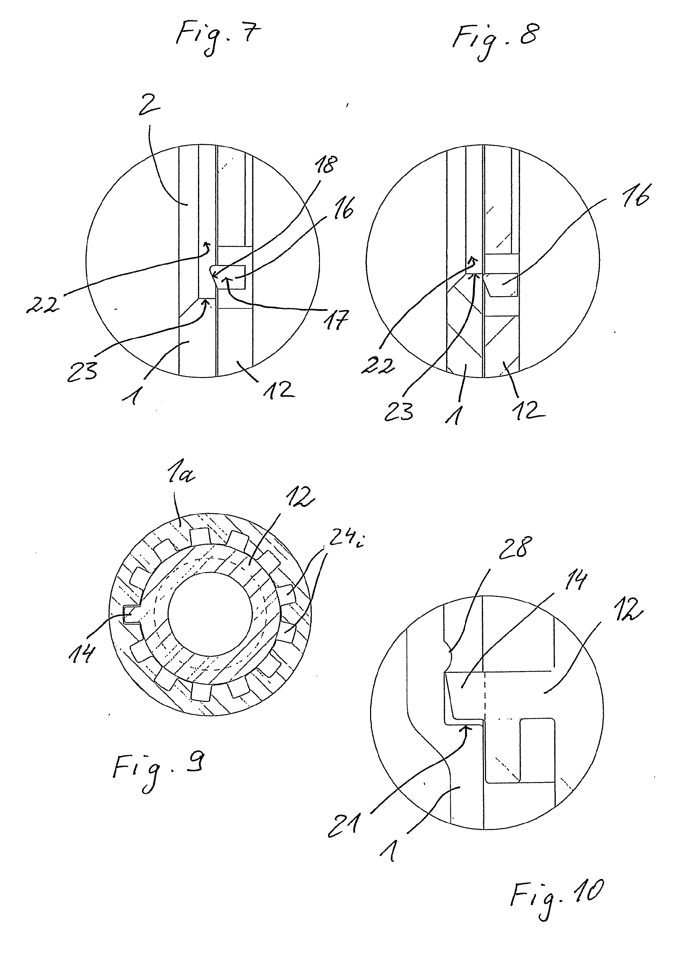

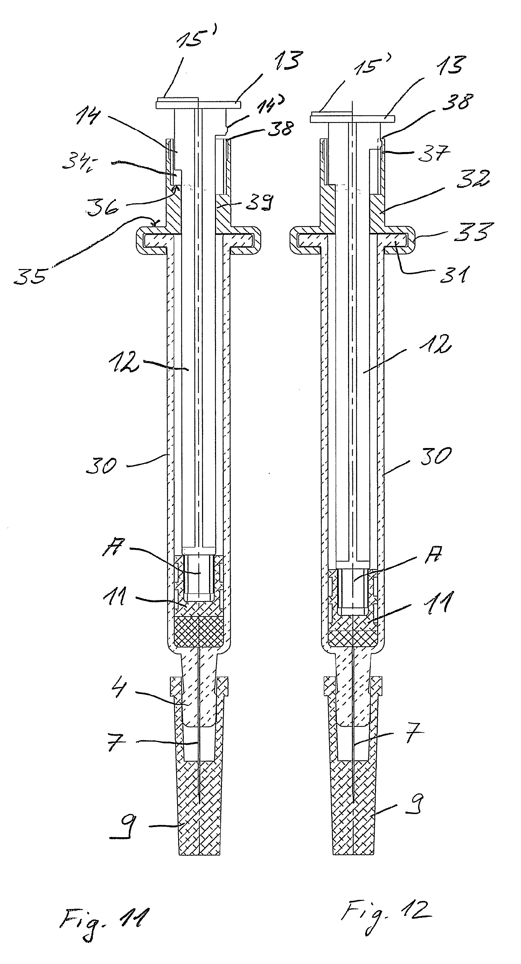

In Figur 7 ist der Bereich, in dem im Ausgangszustand des Geräts der Dosierblockiereingriff besteht, vergrößert im Detail dargestellt. Das Dosierblockierelement 16 ragt über eine äußere Umfangsfläche des Dosier- und Förderglieds 12 radial nach außen in das Dosisanzeigefenster 2 hinein. Es ist am Dosier- und Förderglied 12 so angeordnet, dass es in Bezug auf die Vortriebsrichtung unmittelbar hinter einer vorderen Stirnwand 23 des Fensters 2 in dieses hinein vorsteht und somit dieser Stirnwand 23 axial gegenüberliegt. Für den Dosierblockiereingriff, der im Ausführungsbeispiel aufgrund der Dosierdrehbeweglichkeit des Dosier- und Förderglieds 12 als Verdrehblockiereingriff gebildet ist, weist das Dosierblockierelement 16 in Umfangsrichtung um die Achse A, die Rotationsachse des Dosier- und Förderglieds 12, solch eine Breite auf, dass es wie in Figur 1 erkennbar das Fenster 2 in Umfangsrichtung nahezu vollständig ausfüllt, im Dosierblockiereingriff also in Umfangsrichtung keine "Luft" hat und quasi in enger Passung in beide Drehrichtungen den von den beiden Seitenwänden des Fensters 2 gebildeten Verdrehanschlägen 22 gegenüberliegt. Die in die beiden Drehrichtungen der Drehbeweglichkeit wirksamen Anschlagflächen des Dosierblockierelements 16 sind mit dem Bezugszeichen 17 versehen.

Das Dosierblockierelement 16 weist über die beiden Verdrehanschlagflächen 17 hinaus eine in Bezug auf die Achse A geneigte axiale Führungsfläche 18 auf, die mit der ihr axial gegenüberliegenden Stirnfläche 23 des Dosisanzeigefensters 2 zusammenwirkt. Wird auf das Dosier- und Förderglied 12 eine ausreichend große axiale Druckkraft ausgeübt, nämlich durch Druck auf das Bedienelement 13, wird das Dosierblockierglied 16 mit seiner geneigten Führungsfläche 18 axial gegen die Stirnfläche 23 gedrückt. Das Dosierblockierglied 16 ist in Bezug auf die Achse A radial elastisch nachgiebig, so dass es aufgrund der geneigten Führungsfläche 18 an der Stirnfläche 23 abgleitet und dabei nach radial innen und somit aus dem Dosierblockiereingriff mit dem in Bezug auf die Drehrichtung linken und rechten Verdrehanschlag 22 gelangt. Die Stirnfläche bildet einen Deblockieranschlag 23.

In Figur 8 ist der Zustand dargestellt, in dem das Dosierblockierelement 16 gerade den Deblockieranschlag 23 in die Vortriebsrichtung passiert hat, so dass das Dosier- und Förderglied 12 vom Dosierblockiereingriff frei und um die Achse A drehbeweglich ist.

Figur 9 zeigt das Verabreichungsgerät in einem Querschnitt im Bereich des Dosierabschnitts 1a während der Ausschüttung des Medikaments. Das Dosierelement 14 wird von einem der gleichmäßig um die Achse A im Dosierabschnitt 1a geformten Dosierelemente 24ᵢ axial geführt, so dass das Dosier- und Förderglied 12 während seines Ausschütthubs die bei der Dosisauswahl eingestellte Drehwinkelposition zwangsweise beibehält. Die Figuren 6 und 10 zeigen das Verabreichungsgerät nach vollständiger Ausschüttung der eingestellten Dosis, beispielhaft wurde die Maximaldosis ausgeschüttet.

Figur 6 zeigt das Verabreichungsgerät in einem Längsschnitt, der um einige Winkelgrade um die Achse A von den Längsschnitten der Figuren 4 und 5 versetzt ist. In den Figuren 4 und 5 ist das axial längste der Dosierelemente 24ᵢ verdeckt, während der Längsschnitt der Figur 6 sich durch dieses Dosierelement 24ᵢ erstreckt. Der Ausschütthub wird am in Vortriebsrichtung vorderen Ende jedes der Dosierelemente 24ᵢ durch den jeweiligen Ausschüttanschlag 21 beendet, indem das Dosierelement 14 gegen den jeweiligen Ausschüttanschlag 21 des Gehäuses 1 in die Vortriebsrichtung auf Anschlag gelangt, wie Figur 6 dies zeigt. Jedes der Dosierelemente 24ᵢ weist an seinem in Vortriebsrichtung vorderen Ende einen Ausschüttanschlag 21 auf, wobei die Ausschüttanschläge 21 den unterschiedlichen Dosen entsprechend auf unterschiedlichen axialen Höhen geformt sind.

Das Verabreichungsgerät weist als weitere Besonderheit eine Rückhalteeinrichtung für das Dosier- und Förderglied 12 auf. Die Rückhalteeinrichtung sorgt dafür, dass das Dosier- und Förderglied 12 nach vollständiger Ausführung des Ausschütthubs in der dann eingenommenen Ausschüttposition verbleibt. Sie umfasst Rückhalteelemente 28, die den Dosierelementen 24ᵢ zugeordnet sind, nämlich jeweils eines der Rückhalteelemente 28 für jedes der Dosierelemente 24ᵢ. In Figur 6 sind das Rückhalteelement 28 des axial längsten Dosierelements 24ᵢ und das Rückhalteelement 28 des axial kürzesten Dosierelements 24ᵢ zu erkennen. Die Rückhalteelemente 28 werden vom Gehäuse 1 gebildet und sind beispielhaft an dessen Innenmantelfläche als nach radial innen vorragende Rückhaltenocken geformt. Der Ausschüttanschlag 14, respektive das Dosierelement 14, ist an einer in die Vortriebsrichtung weisenden Vorderseite in Bezug auf die Achse A geneigt, so dass er/es in die Vortriebsrichtung über das Rückhalteelement 28 des jeweiligen Dosierelements 24ᵢ gleiten kann. Die Rückseite des Ausschüttanschlags 14, respektive Dosierelements 14, weist zumindest im Wesentlichen orthogonal zur Achse A, so dass er/es im Zusammenwirken mit dem jeweiligen Rückhalteelement 28 das Dosier- und Förderglied 12 in dessen Ausschüttposition hält, entweder gegen eine Bewegung entgegen der Vortriebsrichtung blockiert oder eine derartige Rückziehbewegung zumindest behindert. Dies sorgt dafür, dass das Verabreichungsgerät nicht unbedacht zweckfremd für eine weitere Ausschüttung verwendet werden kann, sollte die eingestellte Dosis kleiner als die im Reservoir 5 im Ausgangszustand befindliche Medikamentendosis sein.

Nachfolgend wird die Funktionsweise des Verabreichungsgeräts im Zusammenhang erläutert:

Der Patient erhält das Gerät im Ausgangszustand der Figur 1. Zur Verabreichung schließt er die Injektionsnadel 7 an das Reservoir 5 an, indem er den Nadelhalter 8 auf den Verbindungsabschnitt 4 steckt oder schraubt. Diesen Zustand zeigt Figur 4. Der Patient muss lediglich noch die Nadelkappe abziehen, die in Figur 4 die Injektionsnadel 7 abdeckt.

Vor der Verabreichung muss das Reservoir 5 entlüftet werden. Das Verabreichungsgerät erzwingt diesen Primingschritt, da erst nach dem Primen die zu verabreichende Dosis eingestellt werden kann. Das beim Primen ausgestoßene Volumen ist vorgegeben, nämlich durch den axialen lichten Abstand, den der Ausschüttanschlag 14 vom Priminganschlag 25 aufweist. Ein vorzeitiges Einstellen der Dosis wird durch den Dosierblockiereingriff zwischen dem Dosierblockierelement 16 und dem Verdrehanschlag 22 des Gehäuses 1 verhindert. Der Dosierblockiereingriff zwischen 16 und 21 ist insbesondere auch in Figur 1 erkennbar.

In diesem Ausgangszustand liefert die Dosisanzeige dem Patienten den Hinweis, dass er ein Primen ausführen muss und wie dies geschieht. Im Dosisanzeigefenster 2 erscheint die in Figur 1 erkennbare Anzeige, ein Piktogramm, eines in die Vortriebsrichtung weisenden Richtungspfeils und eines "P" für "Primen". Der Patient entlüftet das Reservoir 5 durch Ausübung einer axialen Druckkraft auf das Bedienelement 13. Unter der Druckkraft gibt das elastisch nachgiebige Dosierblockierelement 16 radial nach innen nach, indem seine Führungsfläche 18 über den Deblockieranschlag 23 des Gehäuses 1 gleitet und das Dosierblockierelement 16 durch dieses Abgleiten nach radial innen aus dem Dosierblockiereingriff bewegt wird. Der Vorgang ist am deutlichsten aus den Figuren 7 und 8 ersichtlich. Der Priminghub wird durch den Priminganschlag 25 begrenzt, d. h. der Priminghub ist beendet, sobald der Ausschüttanschlag 14 gegen den Priminganschlag 25 auf Anschlag gelangt.

Figur 5 zeigt das Verabreichungsgerät unmittelbar nach Ausführung des Priminghubs. Der Dosierblockiereingriff ist gelöst. In diesem Zustand kann durch Drehen des Dosier- und Förderglieds 12 um die Achse A die zu verabreichende Dosis eingestellt werden, indem das Dosier- und Förderglied 12 um die Achse A in eine Drehwinkelposition gedreht wird, in der der Ausschüttanschlag 14, der beim Dosieren als Dosierelement 14 wirkt, in axialer Flucht mit demjenigen der Dosierelemente 24ᵢ ist, das seiner axialen Länge nach der zu verabreichenden Dosis entspricht. Im Dosisanzeigefenster 2 ist die eingestellte Dosis ablesbar (Figur 2).

Nach dem Einstellen der Dosis setzt der Patient das Verabreichungsgerät mit der Injektionsnadel 7 an der gewünschten Einstechstelle an und sticht die Injektionsnadel 7 in und unter die Haut. Nach dem Einstechen wird die eingestellte Dosis ausgeschüttet, indem der Patient durch Ausübung einer axialen Druckkraft auf das Bedienelement 13 das Dosier- und Förderglied 12 in die Vortriebsrichtung bewegt. Das Dosier- und Förderglied 12 drückt dabei gegen den Kolben 11 und diesen im Reservoir 5 in Richtung auf den Auslass vor, bis das Dosier- und Förderglied 12 mit seinem Ausschüttanschlag 14 gegen den Ausschüttanschlag 21 stößt, der dem ausgewählten Dosierelement 24ᵢ zugeordnet ist. Der Ausschütthub des Dosier- und Förderglieds 12 und des Kolbens 11, also der Fördereinrichtung 10, entspricht der axialen Länge des ausgewählten Dosierelements 24ᵢ. Figur 9 zeigt das Verabreichungsgerät in einem Querschnitt während des Ausschütthubs mit dem Dosierelement 14 bzw. Ausschüttanschlag 14 im Führungseingriff mit dem ausgewählten Dosierelement 24ᵢ.

Kurz vor dem Ende des Ausschütthubs überfährt der Ausschüttanschlag 14 (Dosierelement 14) das dem ausgewählten Dosierelement 24ᵢ zugeordnete Rückhalteelement 28. Das hierbei entstehende Klick-Geräusch vermittelt dem Patienten ein hörbares und auch fühlbares Signal dafür, dass der Ausschütthub vollständig ausgeführt wurde. Die Figuren 6 und 10 zeigen das Verabreichungsgerät nach Ausführung des vollständigen Ausschütthubs, Figur 10 zeigt den Bereich um das Rückhalteelement 28 in einem vergrößerten Detail. Das Dosier- und Förderglied 12 befindet sich nun im Rückhalteeingriff, der ein Zurückziehen des Dosier- und Förderglieds 12 gegen die Vortriebsrichtung verhindert oder zumindest stark behindert. Wie in Figur 3 erkennbar, liefert die Dosisanzeige durch das Fenster 2 hindurch eine Blindanzeige. In einer Weiterentwicklung kann die Dosisanzeige 15 um ein Piktogramm oder eine Farbmarkierung oder auch einen Text ergänzt werden, um dem Patienten eine positive Rückmeldung des Inhalts zu liefern, dass die eingestellte Dosis vollständig ausgeschüttet wurde. Durch das Reservoirfenster 3 kann der Patient den Kolben 11 erkennen. Im Ergebnis wird ihm somit auch visuell signalisiert, dass die Ausschüttung vollständig durchgeführt wurde.

### Bezugszeichen:

- 1: Gehäuse
- 1a: Dosierabschnitt
- 2: Dosisanzeigefenster
- 3: Reservoirfenster
- 4: Verbindungsabschnitt
- 5: Reservoir, Karpule
- 6: Septum
- 7: Injektionsnadel
- 8: Nadelhalter
- 10: Fördereinrichtung
- 11: Förderglied, Kolben
- 12: Dosier- und Förderglied, Dosierglied, Förderglied
- 13: Bedienelement
- 14: Dosierelement, Ausschüttanschlag
- 15: Dosisskala
- 16: Dosierblockierelement
- 17: Verdrehgegenanschlag
- 18: Führungsfläche
- 19: Dosierraststruktur
- 20: -
- 21: Ausschüttanschlag
- 22: Verdrehanschlag
- 23: Deblockieranschlag
- 24ᵢ: Dosierelement, Führung
- 25: Priminganschlag
- 26: -
- 27: -
- 28: Rückhalteelement
- 29: Dosierrastelement

- A: Vortriebsachse, Rotationsachse

## Patentansprüche

1. Verabreichungsgerät, vorzugsweise Injektionsgerät, umfassend
(a) ein Gehäuse (1),
(b) ein von dem Gehäuse (1) gebildetes oder aufgenommenes Reservoir (5) für ein flüssiges Medikament,
(c) ein Dosierglied (12-19), das zum Einstellen einer zu verabreichenden Dosis des Medikaments relativ zum Gehäuse (1) eine Dosierbewegung ausführen kann,
(d) und eine Fördereinrichtung (10) mit einem Förderglied (12-14) zum Ausschütten der eingestellten Dosis
(e) wobei das Dosierglied (12-19) in einem Ausgangszustand des Verabreichungsgeräts in einem lösbaren Dosierblockiereingriff (16, 22) in Richtung der Dosierbewegung blockiert ist
(f) und am Ende einer der Entlüftung des Reservoirs (5) dienenden Primingbewegung des Förderglieds (12-14) von der Blockierung freikommt,
(g) und wobei das Förderglied (12-14) für die Ausführung der Primingbewegung und zum Ausschütten der eingestellten Dosis relativ zu dem Reservoir (5) in eine Vortriebsrichtung beweglich ist,
**dadurch gekennzeichnet, dass**
(h) das Dosierglied (12-19) durch eine in die Vortriebsrichtung wirkende Kraft gegen eine elastische Rückstellkraft in die Vortriebsrichtung aus dem Dosierblockiereingriff (16, 22) bewegbar ist, wobei die Rückstellkraft von einem quer zur Vortriebsrichtung elastisch nachgiebigen Schnapper (16) erzeugt wird.

2. Verabreichungsgerät nach dem vorhergehenden Anspruch, **dadurch gekennzeichnet, dass** das Dosierglied (12-19) für die Ausführung der Dosierbewegung relativ zum Gehäuse (1) um eine Rotationsachse (A) drehbeweglich und in dem Dosierblockiereingriff (16, 22) in Umfangsrichtung um die Rotationsachse (A) gegen einen Verdrehanschlag (22) auf Anschlag ist, wobei das Dosierglied (12-19) vorzugsweise längs des Verdrehanschlags (22) axial aus dem Dosierblockiereingriff (16, 22) mit dem Verdrehanschlag (22) beweglich ist.

3. Verabreichungsgerät nach dem vorhergehenden Anspruch, **dadurch gekennzeichnet, dass** das Gehäuse (1), vorzugsweise eine Seitenwand eines im Gehäuse (1) geformten Fensters (2), den Verdrehanschlag (22) bildet.

4. Verabreichungsgerät nach einem der vorhergehenden Ansprüche, **dadurch gekennzeichnet, dass** das Dosierglied (12-19) mit dem Förderglied (12-14) so gekoppelt ist, dass es durch die Primingbewegung des Förderglieds (12-14) aus dem Dosierblockiereingriff (16, 22) gelangt, vorzugsweise die Primingbewegung des Förderglieds (12-14) mitmacht.

5. Verabreichungsgerät nach einem der vorhergehenden Ansprüche, **dadurch gekennzeichnet, dass** der Schnapper (16) am Dosierglied (12-19) geformt ist.

6. Verabreichungsgerät nach einem der vorhergehenden Ansprüche, **dadurch gekennzeichnet, dass** das Förderglied (12-14) am Ende der Primingbewegung in einen Anschlagkontakt mit einem vorzugsweise vom Gehäuse (1) gebildeten Priminganschlag (25) gelangt, der die Primingbewegung begrenzt, wobei der Priminganschlag (25) vorzugsweise ein Axialanschlag ist, gegen den das Förderglied (12-14) in die Vortriebsrichtung in den Anschlagkontakt gelangt.

7. Verabreichungsgerät nach dem vorhergehenden Anspruch, **dadurch gekennzeichnet, dass** das Dosierglied (12-19) bei im Anschlagkontakt mit dem Priminganschlag (25) befindlichem Förderglied (12-14) die Dosierbewegung ausführen kann, vorzugsweise relativ zum Gehäuse (1) um eine in die Vortriebsrichtung weisende Rotationsachse (A) drehbeweglich ist.

8. Verabreichungsgerät nach einem der vorhergehenden Ansprüche, **dadurch gekennzeichnet, dass** das Förderglied (12-14) bei dem Ausschütten der eingestellten Dosis mit einem vorzugsweise vom Gehäuse (1) gebildeten Rückhalteelement (28) in einen Rückhalteeingriff (14, 28) gelangt, der eine Bewegung des Förderglieds (12-14) gegen die Vortriebsrichtung blockiert oder zumindest erschwert, wobei das Förderglied (12-14) in dem Rückhalteeingriff gegen die Vortriebsrichtung vorzugsweise in einem Anschlagkontakt mit dem Rückhalteelement (28) ist.

9. Verabreichungsgerät nach einem der vorhergehenden Ansprüche in Kombination mit Anspruch 2, **dadurch gekennzeichnet, dass**
(i) das Dosierglied (12-19) für die Auswahl der Dosis mit einer Dosierstruktur (1a) des Gehäuses (1) zusammenwirkt,
(ii) eines aus Dosierglied (12-19) und Dosierstruktur (1a) ein erstes Dosierelement (14) und das andere um die Rotationsachse (A) verteilt mehrere zweite Dosierelemente (24ᵢ) aufweist,
(iii) jedes der zweiten Dosierelemente (24ᵢ) einer bestimmten Dosis entspricht, die sich von der Dosis jedes anderen der zweiten Dosierelemente (24ᵢ) unterscheidet,
(iv) entweder das erste Dosierelement (14) ein Eingriffselement und die zweiten Dosierelemente (24ᵢ) axial erstreckte Führungen oder das erste Dosierelement eine axial erstreckte Führung und die zweiten Dosierelemente Eingriffselemente sind
(v) und das Dosierglied (12-19) durch das Einstellen der Dosis in eine Drehwinkelposition bewegt wird, in der das erste Dosierelement (14) mit demjenigen der zweiten Dosierelemente (24ᵢ) in einen Führungseingriff gelangt, das der eingestellten Dosis entspricht.

10. Verabreichungsgerät nach einem der vorhergehenden Ansprüche, umfassend ein Betätigungselement (13) für eine manuelle Betätigung des Dosierglieds (12-19) oder des Förderglieds (12-14), wobei das Betätigungselement (13) axial oder rotatorisch relativ zu dem Förderglied (12-14) oder dem Dosierglied (12-19) vorzugsweise unbeweglich ist.

11. Verabreichungsgerät nach einem der vorhergehenden Ansprüche, **dadurch gekennzeichnet, dass** das Dosierglied (12-19) axial oder rotatorisch relativ zu dem Förderglied (12-14) unbeweglich, vorzugsweise in einem Stück mit dem Förderglied (12-14) geformt ist.

12. Verabreichungsgerät nach einem der vorhergehenden Ansprüche, umfassend eine Dosisanzeige (2, 15) mit einer Dosisskala (15) und einem Marker (2), dessen Position relativ zur Dosisskala (15) die eingestellte Dosis anzeigt, wobei vorzugsweise das Dosierglied (12-19) die Dosisskala (15) aufweist und der Marker (2) vorzugsweise in einem Schalenbereich des Gehäuses (1) als Fenster geformt ist.

13. Verabreichungsgerät nach einem der vorhergehenden Ansprüche, **dadurch gekennzeichnet, dass** das Gehäuse (1) einen Ausschüttanschlag (21) bildet, gegen den das Förderglied (12-14) bei dem Ausschütten in Anschlagkontakt gelangt, sobald die eingestellte Dosis ausgeschüttet ist.

14. Verabreichungsgerät nach einem der vorhergehenden Ansprüche, **dadurch gekennzeichnet, dass** das Reservoir (5) das flüssige Medikament enthält, das Medikament nicht konserviert und das Reservoir (5) steril geschlossen ist, wobei das Reservoir (5) vorzugsweise eine Karpule (5) oder Spritze ist.

15. Verabreichungsgerät nach einem der vorhergehenden Ansprüche, **dadurch gekennzeichnet, dass** das Reservoir (5) im steril geschlossenen Zustand das Medikament in einer Menge enthält, die höchstens so groß wie eine Maximaldosis ist, in der das Medikament in einer einzigen Injektion verabreicht wird.

16. Verabreichungsgerät nach einem der vorhergehenden Ansprüche, **dadurch gekennzeichnet, dass** das Verabreichungsgerät oder ein das Reservoir (5) enthaltender oder bildender Teil des Verabreichungsgeräts ein Einwegartikel für nur eine einzige Verabreichung, vorzugsweise Injektion, ist.

17. Verabreichungsgerät nach einem der Ansprüche 14 bis 16, **dadurch gekennzeichnet, dass** das flüssige Medikament ein Medikament auf der Basis von FSH oder einer FSH Variante ist.

18. Verabreichungsgerät nach einem der vorhergehenden Ansprüche, **dadurch gekennzeichnet, dass** das Förderglied (12-14) eine das Medikament ausschüttende Ausschüttbewegung ausführt und nach einer einzigen vollständigen Verabreichung, vorzugsweise Injektion, in der das gesamte in dem Reservoir enthaltene Medikament oder die mittels des Dosierglieds (12-19) eingestellte Dosis ausgeschüttet wird, automatisch in einen Rückhalteeingriff (14, 28) gelangt, der eine weitere Ausschüttung verhindert oder zumindest behindert.

19. Verabreichungsgerät nach einem der vorhergehenden Ansprüche, umfassend eine Injektionsnadel, die mit dem im Reservoir befindlichen Medikament in Fluidverbindung steht und gegen die äußere Umgebung mittels eines Dichtelements steril geschlossen ist.

20. Verabreichungsgerät nach einem der Ansprüche 1 bis 18, **dadurch gekennzeichnet, dass** ein durchstechbares Septum (6) das Reservoir (5) luftdicht und steril schließt und das Gehäuse (1) oder das Reservoir einen Verbindungsabschnitt (4) für den Anschluss einer bei dem Anschluss das Septum (6) durchstechenden Injektionsnadel (7) aufweist.

21. Verabreichungsgerät nach einem der vorhergehenden Ansprüche und wenigstens einem der folgenden Merkmale:
(i) das Reservoir wird von einer Karpule (5) oder einer Spritze gebildet, in der ein als Stopfen gebildeter Kolben (11) in Richtung auf einen Auslass des Reservoirs (5) beweglich aufgenommen ist, um das Medikament auszuschütten;
(ii) das Reservoir umfasst einen Kolben (11), der das Reservoir (5) steril verschließt und in Richtung auf einen Auslass des Reservoirs (5) beweglich ist, um das Medikament auszuschütten.

## Claims

1. An administration device, preferably an injection device, comprising
(a) a housing (1),
(b) a reservoir (5) for a liquid drug, which is formed or received by the housing (1),
(c) a dosing member (12-19) which can perform a dosing movement relative to the housing (1) for setting a dose to be administered of the drug, and
(d) a conveyor device (10) having a conveyor member (12-14) for dispensing the set dose,
(e) wherein in an initial condition of the administration device the dosing member (12-19) is blocked in a releasable dosing blocking engagement (16, 22) in the direction of the dosing movement
(f) and at the end of a priming movement of the conveyor member (12-14), serving for venting of the reservoir (5), comes free from the blocking action,
(g) and wherein the conveyor member (12-14) is moveable relative to the reservoir (5) in an advancing direction for performing the priming movement and for dispensing the set dose,
**characterised in that**
(h) the dosing member (12-19) is moveable in the advancing direction out of the dosing blocking engagement (16, 22) by a force acting in the advancing direction against a resilient return force, wherein the return force is produced by a snap catch (16) which is elastically yielding transversely relative to the advancing direction.

2. An administration device according to the preceding claim **characterised in that** the dosing member (12-19) is rotationally moveable about an axis of rotation (A) for performing the dosing movement relative to the housing (1) and in the dosing blocking engagement (16, 22) is in an abutment condition in the peripheral direction about the axis of rotation (A) against a rotational abutment (22), wherein the dosing member (12-19) is preferably moveable along the rotational abutment (22) axially out of the dosing blocking engagement (16, 22) with the rotational abutment (22).

3. An administration device according to the preceding claim **characterised in that** the housing (1), preferably a side wall of a window (2) formed in the housing (1), forms the rotational abutment (22).

4. An administration device according to one of the preceding claims **characterised in that** the dosing member (12-19) is so coupled to the conveyor member (12-14) that due to the priming movement of the conveyor member (12-14) it comes out of the dosing blocking engagement (16, 22), and preferably also performs the priming movement of the conveyor member (12-14).

5. An administration device according to one of the preceding claims **characterised in that** the snap catch (16) is formed on the dosing member (12-19).

6. An administration device according to one of the preceding claims **characterised in that** at the end of the priming movement the conveyor member (12-14) comes into abutting contact with a priming abutment (25) which is preferably formed by the housing (1) and which delimits the priming movement, wherein the priming abutment (25) is preferably an axial abutment, against which the conveyor member (12-14) passes into the condition of abutting contact in the advancing direction.

7. An administration device according to the preceding claims **characterised in that** with the conveyor member (12-14) in abutting contact with the priming abutment (25) the dosing member (12-19) can perform the dosing movement, preferably being rotationally moveable relative to the housing (1) about an axis of rotation (A) facing in the advancing direction.

8. An administration device according to one of the preceding claims **characterised in that** upon dispensing of the set dose the conveyor member (12-14) comes into retaining engagement (14, 28) with a retaining element (28) preferably formed by the housing (1), which retaining engagement blocks or at least renders difficult a movement of the conveyor member (12-14) in opposite relationship to the advancing direction, wherein the conveyor member (12-14) is preferably in abutting contact with the retaining element (28) in the retaining engagement in opposite relationship to the advancing direction.

9. An administration device according to one of the preceding claims in combination with claim 2 **characterised in that**
(i) the dosing member (12-19) cooperates with a dosing structure (1a) of the housing (1) for selecting the dose,
(ii) one of the dosing member (12-19) and the dosing structure (1a) has a first dosing element (14) and the other has a plurality of second dosing elements (24) distributed around the axis of rotation (A),
(iii) each of the second dosing elements (24ᵢ) corresponds to a given dose which differs from the dose of each other of the second dosing elements (24ᵢ),
(iv) either the first dosing element (14) is an engagement element and the second dosing elements (24ᵢ) are axially extended guides or the first dosing element is an axially extended guide and the second dosing elements are engagement elements
(v) and the dosing member (12-19) is moved by setting of the dose into a rotational angular position in which the first dosing element (14) comes into a guide engagement with that one of the second dosing elements (24ᵢ) that corresponds to the set dose.

10. An administration device according to one of the preceding claims including an actuating element (13) for manual actuation of the dosing member (12-19) or the conveyor member (12-14), wherein the actuating element (13) is preferably immovable axially or rotationally relative to the conveyor member (12-14) or the dosing member (12-19).

11. An administration device according to one of the preceding claims **characterised in that** the dosing member (12-19) is immovable axially or rotationally relative to the conveyor member (12-14) and is preferably formed in one piece with the conveyor member (12-14).

12. An administration device according to one of the preceding claims including a dose display (2, 15) having a dose scale (15) and a marker (2) whose position relative to the dose scale (15) shows the set dose, wherein preferably the dosing member (12-19) has the dose scale (15) and the marker (2) is preferably formed as a window in a shell region of the housing (1).

13. An administration device according to one of the preceding claims **characterised in that** the housing (1) forms a dispensing abutment (21) against which the conveyor member (12-14) comes into abutting contact in the dispensing operation as soon as the set dose is dispensed.

14. An administration device according to one of the preceding claims **characterised in that** the reservoir (5) contains the liquid drug, the drug is non-preserved and the reservoir (5) is closed in a sterile condition, wherein the reservoir (5) is preferably a carpule (5) or syringe.

15. An administration device according to one of the preceding claims **characterised in that** the reservoir (5) in the sterile closed condition contains the drug in an amount which is at most as large as a maximum dose in which the drug is administered in a single injection.

16. An administration device according to one of the preceding claims **characterised in that** the administration device or a part of the administration device which forms or includes the reservoir (5) is a disposable item for only a single administration, preferably injection.

17. An administration device according to one of claims 14 to 16 **characterised in that** the liquid drug is a drug on the basis of FSH or FSH variant.

18. An administration device according to one of the preceding claims **characterised in that** the conveyor member (12-14) performs a dispensing movement for dispensing the drug and after a single complete administration, preferably injection, in which all the drug contained is the reservoir or the dose set by means of the dosing member (12-19) is dispensed automatically passes into a retaining engagement (14, 28) which a prevents or at least impedes a further dispensing operation.

19. An administration device according to one of the preceding claims including an injection needle in fluid communication with the drug in the reservoir and closed in a sterile condition in relation to the outside environment by means of a sealing element.

20. An administration device according to one of claims 1 to 18 **characterised in that** a puncturable septum (6) air-tightly and sterilely closes the reservoir (5) and the housing (1) or the reservoir has a connecting portion (4) for the connection of an injection needle (7) which punctures the septum (6) when connected.

21. An administration device according to one of the preceding claims and having at least one of the following features:
(i) the reservoir is formed by a carpule (5) or a syringe in which a plunger (11) in the form of a stopper is movably accommodated in a direction towards an outlet of the reservoir (5) to dispense the drug; and
(ii) the reservoir includes a plunger (11) which sterilely closes the reservoir (5) and is moveable in a direction towards an outlet of the reservoir (5) to dispense the drug.

## Revendications

1. Appareil d'administration, de préférence appareil d'injection comprenant
(a) un boîtier (1),
(b) un réservoir (5) reçu ou formé par le boîtier (1) pour un médicament liquide,
(c) un organe de dosage (12-19) qui peut réaliser un mouvement de dosage par rapport au boîtier (1) pour le réglage d'une dose à administrer du médicament,
(d) et un dispositif d'avance (10) avec un organe d'avance (12-14) pour déverser la dose réglée,
(e) l'organe de dosage (12-19), dans un état initial de l'appareil d'administration, étant bloqué dans la direction du mouvement de dosage dans un engagement de blocage de dosage (16, 22) libérable,
(f) et étant libéré du blocage à la fin d'un mouvement d'amorçage de l'organe d'avance (12-14) servant à l'aération du réservoir (5),
(g) et l'organe d'avance (12-14) étant mobile par rapport au réservoir (5) dans une direction d'avance pour la réalisation du mouvement d'amorçage et pour le déversement de la dose réglée,
**caractérisé en ce que**
(h) l'organe de dosage (12-19) est mobile dans la direction d'avance hors de l'engagement de blocage de dosage (16, 22) par une force agissant dans la direction d'avance à l'encontre d'une force de rappel élastique, la force de rappel étant générée par un loquet (16) flexible élastiquement transversalement à la direction d'avance.

2. Appareil d'administration selon la revendication précédente, **caractérisé en ce que** l'organe de dosage (12-19) est mobile en rotation par rapport au boîtier (1) autour d'un axe de rotation (A) pour la réalisation du mouvement de dosage et est en butée dans l'engagement de blocage de dosage (16, 22) dans la direction circonférentielle autour de l'axe de rotation (A) contre une butée de rotation (22), l'organe de dosage (12-19) étant de préférence mobile le long de la butée de rotation (22) axialement hors de l'engagement de blocage de dosage (16, 22) avec la butée de rotation (22).

3. Appareil d'administration selon la revendication précédente, **caractérisé en ce que** le boîtier (1), de préférence une paroi latérale d'une fenêtre (2) formée dans le boîtier (1), forme la butée de rotation (22).

4. Appareil d'administration selon l'une quelconque des revendications précédentes, **caractérisé en ce que** l'organe de dosage (12-19) est couplé à l'organe d'avance (12-14) de sorte qu'il parvienne par le mouvement d'amorçage de l'organe d'avance (12-14) hors de l'engagement de blocage de dosage (16, 22), de préférence participe au mouvement d'amorçage de l'organe d'avance (12-14).

5. Appareil d'administration selon l'une quelconque des revendications précédentes, **caractérisé en ce que** le loquet (16) est formé sur l'organe de dosage (12-19).

6. Appareil d'administration selon l'une quelconque des revendications précédentes, **caractérisé en ce que** l'organe de dosage (12-14) parvient à la fin du mouvement d'amorçage dans un contact de butée avec une butée d'amorçage (25) formée de préférence par le boîtier (1), laquelle délimite le mouvement d'amorçage, la butée d'amorçage (25) étant de préférence une butée axiale, contre laquelle l'organe d'avance (12-14) parvient dans la direction d'avance dans le contact de butée.

7. Appareil d'administration selon la revendication précédente, **caractérisé en ce que** l'organe de dosage (12-19) peut réaliser le mouvement de dosage en cas d'organe d'avance (12-14) se trouvant en contact de butée avec la butée d'amorçage (25), de préférence est mobile en rotation par rapport au boîtier (1) autour d'un axe de rotation (A) orienté dans la direction d'avance.

8. Appareil d'administration selon l'une quelconque des revendications précédentes, **caractérisé en ce que** l'organe d'avance (12-14) parvient lors du déversement de la dose réglée dans un engagement de retenue (14, 28) avec un élément de retenue (28) formé de préférence par le boîtier (1), lequel élément de retenue bloque ou au moins s'oppose à un mouvement de l'organe d'avance (12-14) dans la direction opposée à la direction d'avance, l'organe d'avance (12-14) étant dans l'engagement de retenue dans la direction opposée à la direction d'avance, de préférence dans un contact de butée avec l'élément de retenue (28).

9. Appareil d'administration selon l'une quelconque des revendications précédentes en combinaison avec la revendication 2, **caractérisé en ce que**
(i) l'organe de dosage (12-19) coopère pour la sélection de la dose avec une structure de dosage (1a) du boîtier (1),
(ii) l'un de l'organe de dosage (12-19) et de la structure de dosage (1a) présente un premier élément de dosage (14) et l'autre présente, répartis autour de l'axe de rotation (A), plusieurs seconds éléments de dosage (24ᵢ),
(iii) chacun des seconds éléments de dosage (24ᵢ) correspondant à une dose déterminée qui se distingue de la dose de chaque autre des seconds éléments de dosage (24ᵢ),
(iv) le premier élément de dosage (14) est un élément d'engagement et les seconds éléments de dosage (24ᵢ) sont des guidages s'étendant axialement ou le premier élément de dosage est un guidage s'étendant axialement et les seconds éléments de dosage sont des éléments d'engagement
(v) et l'organe de dosage (12-19) est déplacé par le réglage de la dose dans une position angulaire en rotation, dans laquelle le premier élément de dosage (14) parvient dans un engagement de guidage avec l'élément des seconds éléments de dosage (24ᵢ) qui correspond à la dose réglée.

10. Appareil d'administration selon l'une quelconque des revendications précédentes, comprenant un élément d'actionnement (13) pour un actionnement manuel de l'organe de dosage (12-19) ou de l'organe d'avance (12-14), l'élément d'actionnement (13) étant de préférence immobile axialement ou rotatif par rapport à l'organe d'avance (12-14) ou l'organe de dosage (12-19).

11. Appareil d'administration selon l'une quelconque des revendications précédentes, **caractérisé en ce que** l'organe de dosage (12-19) est immobile axialement ou en rotation par rapport à l'organe d'avance (12-14), de préférence est formé en une pièce avec l'organe d'avance (12-14).

12. Appareil d'administration selon l'une quelconque des revendications précédentes, comprenant un affichage de dosage (2, 15) avec une échelle de dosage (15) et un marqueur (2), dont la position par rapport à l'échelle de dosage (15) affiche la dose réglée, l'organe de dosage (12-19) présentant de préférence l'échelle de dosage (15) et le marqueur (2) étant de préférence formé dans une zone d'enveloppe du boîtier (1) sous forme de fenêtre.

13. Appareil d'administration selon l'une quelconque des revendications précédentes, **caractérisé en ce que** le boîtier (1) forme une butée de déversement (21), contre laquelle l'organe de d'avance (12-14) parvient en contact de butée lors du déversement dès que la dose réglée est déversée.

14. Appareil d'administration selon l'une quelconque des revendications précédentes, **caractérisé en ce que** le réservoir (5) contient le médicament liquide, ne conserve pas le médicament et le réservoir (5) est fermé de manière stérile, le réservoir (5) étant de préférence une ampoule (5) ou une seringue.

15. Appareil d'administration selon l'une quelconque des revendications précédentes, **caractérisé en ce que** le réservoir (5) contient dans l'état fermé de manière stérile le médicament dans une quantité qui est au maximum aussi grande qu'une dose maximale, dans laquelle le médicament est administré en une seule injection.

16. Appareil d'administration selon l'une quelconque des revendications précédentes, **caractérisé en ce que** l'appareil d'administration ou une partie contenant ou formant le réservoir (5) de l'appareil d'administration est un article à usage unique pour une seule administration, de préférence une injection.

17. Appareil d'administration selon l'une quelconque des revendications 14 à 16, **caractérisé en ce que** le médicament liquide est un médicament à base de FSH ou d'une variante de FSH.

18. Appareil d'administration selon l'une quelconque des revendications précédentes, **caractérisé en ce que** l'organe d'avance (12-14) réalise un mouvement de déversement déversant le médicament et parvient automatiquement après une seule administration complète, de préférence une injection, dans laquelle le médicament entier contenu dans le réservoir ou la dose réglée à l'aide de l'organe de dosage (12-19) est déversé(e), dans un engagement de retenue (14, 28) qui empêche ou au moins s'oppose à un autre déversement.

19. Appareil d'administration selon l'une quelconque des revendications précédentes, comprenant une aiguille d'injection qui est en liaison fluidique avec le médicament se trouvant dans le réservoir et est fermée de manière stérile par rapport à l'environnement extérieur à l'aide d'un élément étanche.

20. Appareil d'administration selon l'une quelconque des revendications 1 à 18, **caractérisé en ce qu'**un septum transperçable (6) ferme de manière étanche à l'air et stérile le réservoir (5) et le boîtier (1) ou le réservoir présente une portion de liaison (4) pour le raccordement d'une aiguille d'injection (7) transperçant le septum (6) lors du raccordement.

21. Appareil d'administration selon l'une quelconque des revendications précédentes et au moins l'une des caractéristiques suivantes :
(i) le réservoir est formé par une ampoule (5) ou une seringue, dans laquelle un piston (11) formé comme un bouchon est reçu de manière mobile en direction d'une sortie du réservoir (5) afin de déverser le médicament ;
(ii) le réservoir comporte un piston (11) qui ferme de manière stérile le réservoir (5) et est mobile en direction d'une sortie du réservoir (5) afin de déverser le médicament.
